# NEW EUROPEAN PATENT SPECIFICATION

(11) **EP 2 986 156 B2**
(45) Date of publication and mention of the opposition decision: **03.06.2020**
(45) Mention of the grant of the patent: 21.12.2016
(21) Application number: 14717146.6
(22) Date of filing: 14.04.2014
(51) Int. Cl.: A23L 33/10, A23L 33/155, A23L 33/19, A61H 1/00, A63B 21/00

(54) **USE OF WHEY PROTEIN IN COMBINATION WITH ELECTRICAL MUSCLE STIMULATION**
VERWENDUNG VON WHEY-PROTEIN IN KOMBINATION MIT ELEKTRISCHER MUSKELSTIMULATION
UTILISATION DE LA PROTÉINE DE LACTOSÉRUM EN COMBINAISON AVEC UNE STIMULATION MUSCULAIRE ÉLECTRIQUE

(30) Priority: 15.04.2013 EP 13163687
(43) Date of publication of application: 24.02.2016
(73) Proprietor: Société des Produits Nestlé S.A., 1800 Vevey (CH)
(72) Inventor: ARIGONI, Fabrizio, Tokyo 102-0084 (JP); BREUILLE, Denis, CH-1010 Lausanne (CH); MORITANI, Toshio, Kyoto 606-8501 (JP); OFFORD CAVIN, Elizabeth, CH-1820 Montreux (CH); VINYES PARES, Gerard, Tokyo 102-0084 (JP)
(74) Representative: Rupp, Christian
(86) International application number: PCT/EP2014/057474
(87) International publication number: WO 2014/170245

(56) References cited:
- WO-A1-99/13738
- WO-A1-2007/143794
- WO-A1-2010/143939
- WO-A1-2012/081971
- WO-A1-2013/021891
- MATTHIAS KRENN, MICHAEL HALLER, MANFRED BIJAK, EWALD UNGER, CHRISTIAN HOFER, HELMUT KERN, AND WINFRIED MAYR: "Safe Neuromuscular Electrical StimulatorDesigned for the Elderly", ARTIFICIAL ORGANS , vol. 35, no. 3 2011, pages 253-256, XP002716097, DOI: 10.1111/j.1525-1594.2011.01217.x Retrieved from the Internet: URL:http://onlinelibrary.wiley.com/doi/10. 1111/j.1525-1594.2011.01217.x/pdf [retrieved on 2013-11-08]
- MICHAEL LEWEK, JENNIFER STEVENS AND LYNN SNYDER-MACKLER: "The use of electrical stimulation to increase quadriceps femoris muscle force in an elderly patient following a total knee arthroplasty", PHYSICAL THERAPY , vol. 81 2001, pages 1565-1571, XP002716098, Retrieved from the Internet: URL:http://www.physther.org/content/81/9/1 565.full.pdf+html [retrieved on 2013-11-04]
- Cruz-Jentoft Alfonso et al.: "Sarcopenia: European consensus on definition and diagnosis; Report of the European Working Group on Sarcopenia in Older People", Age and Ageing, vol. 39, 2010, pages 412-423,
- REESE et al.: "Neuromuscular electrical stimulation and dietary interventions to reduce oxidative stress in a secondary progressive multiple sclerosis patient leads to marked gains in function: a case report", Case Journal, vol. 2, no. 7601, 10 August 2009 (2009-08-10), pages 1-4,
- WALL et al.: "Neuromuscular electrical stimulation increases muscle protein synthesis in elderly type 2 diabetic men", Am J Physiol Endocrinol Metab, vol. 303, 2012, pages 614-623,
- HAMADA et al.: "Electrical stimulation of human lower extremities enhances energy consumption, carbohydrate oxidation, and whole body glucose uptake", J Appl Physiol, vol. 96, no. 3, 2004, pages 911-916,
- KIM et al.: "Dietary implications on mechanisms of sarcopenia: roles of protein,amino acids and antioxidants", J. Nutrit Biochem, vol. 21, 2010, pages 1-13,
- CAGGIANO et al.: "Effects of electrical stimulation or voluntary contraction for strengthening the quadriceps femoris muscles in an aged male population.", J Orthop Sports Phys Ther, vol. 20, no. 1, July 1994 (1994-07), pages 22-28,
- ENGLISH et al.: "Protecting muscle mass and function in older adults during bedrest", Curr Opi Clin Nutr Metab Care, vol. 13, no. 1, 2010, pages 34-39,
- ESMARCK et al.: "Timing of postexercise protein intake is important for muscle hypertrophy with resistance training in elderly humans", J. Phys 2001, vol. 535, no. 1, August 2001 (2001-08), pages 301-311,
- Jog Mate Protein, info from wayback machine (as available on 11-03-2000), https://web. archive.org/web/20000303114846fw_/http://w ww.jogmate.com:80/inside/index.html & Jog Mate Protein composition, info from "nutrition info in D09A. https://web. archive.org/web/20000303114846fw_/http://w ww.jogmate.com:80/inside/index.html ( Behind button "nutrition info" in left window) & Wayback Machine snapshot of website Otsuka Pharmaceutical on JogMate Protein at 28-04-2001 & English machine treanslation of D09C
- FIATARONE et al.: "Exercise Training and Nutritional Supplementation for Physical Frailty in Very Elderly People", The New England Journal of Medicine, vol. 330, no. 25, 1994, pages 1769-1775,
- "National Research Council, Subcommittee on the Tenth Edition of the RDAs", Recommended dietary allowances, 1989, Washington , D.C.
- "Skim milk composition, Reference Manual for U.S. Milk Powders", U.S. Dairy Export Council, 2005, page 41, Arlington, VA
- "Soy Protein Isolate composition", MY PROTEIN NutritionValue.org, 2017, Retrieved from the Internet: URL:https://www.nutritionvalue.org/Soy_pro tein-isolate-nutritional-value.html
- MARZANI et al.: "Antioxidant Supplementation Restores Defective Leucine Stimulation of Protein Synthesis in Skeletal Muscle from Old Rats", J Nutr, vol. 138, 2008, pages 2205-2211,
- "Rice bran oil", Wikipedia, 12 September 2017 (2017-09-12),
- RODACKI et al.: "Fish-oil supplementation enhances the effects of strength training in elderly women", Am J Clin Nutr, vol. 95, 2012, pages 428-436,
- Bettina Cämmerer ; Lothar W. Kroh: "Antioxidant activity of coffee brews", European Food Research and Technology, Springer Berlin Heidelberg, vol. 223, no. 4, 1 February 2006 (2006-02-01), pages 469-474, ISSN: 1438-2385, DOI: 10.1007/s00217-005-0226-4

## Description

### Technical field of the invention

The present invention relates to a composition comprising an amino acid source selected from whey protein, at least one fatty acid and at least one antioxidant being at least one polyphenol for use in combination with electrical muscular stimulation. In particular the present invention relates to the use of a composition comprising an amino acid source selected from whey protein, at least one fatty acid and at least one antioxidant being at least one polyphenol in combination with electrical muscle stimulation for the treatment or prevention of sarcopenia, for reducing the loss of muscle morphology, for increasing the muscle morphology and/or for improving the muscle recovery after muscle atrophy in elderly humans.

### Background of the invention

Most persons will when they become older suffer of loss of skeletal muscle mass and strength due to ageing. This syndrome where the muscle morphology, i.e. the strength, mass, size and function, is reduced according to the increase in age is called sarcopenia. Sarcopenia is characterized by a decrease in the size of the muscle, which causes weakness and frailty and a decrease in muscle functionality. Sarcopenia in elderly persons is often caused by reduced activity of the body which leads to a reduction of muscle morphology. Sarcopenia can be seen as a muscular analogue of osteoporosis, which is loss of bone, also caused by inactivity and counteracted by exercise.

Muscles in elderly are characterized by an anabolic resistance to meals and a reduced muscle protein synthetic response to dietary amino acids, especially branched chain amino acids such as leucine. The anabolic resistance may be worsened by the low physical activity observed in frail elderly. In addition, oxidative stress and/or a low grade inflammation have also been demonstrated to be associated with frailty in the elderly and could further exacerbate this anabolic resistance.

The skeletal muscle is a highly plastic muscle which reduces under different conditions, e.g. under ageing.

Muscle wasting results from an imbalance between protein synthesis and breakdown rates but also from an imbalance between apoptotic and differentiation/regeneration processes. Muscle proteins can be catabolized into free amino acids (AA) that are used to provide substrates to synthesize protein in other organs for the host defence in the case of various pathologies or stressful events. Thus, the main function of skeletal muscle is to provide power and strength for locomotion and posture, but the muscle is also the major reservoir of proteins and amino acids in the body. Consequently, reduction of muscles during ageing impairs the elderly person's movement.

Due to the lessened physical activity and increased longevity of industrialized populations, sarcopenia is emerging as a major health concern to the society. Sarcopenia may even progress to the extent that an elderly person may lose his or her ability to perform daily activities and thus to live independently. An elderly person suffering from sarcopenia might therefore experience an impaired quality of the life.

Hence, there is an unmet need for a beneficially promoting reduction, prevention, or treatment of sarcopenia in elderly people, i.e. reducing the loss of muscle morphology, such as reducing the loss of muscle function, reducing the loss of muscle mass, reducing the loss of muscle size, reducing the loss of muscle strength, increase the muscle function, increase the muscle size, increase the muscle strength or increase the muscle mass. Further, there is a need for strategies such as nutritional strategies to reduce development of sarcopenia in elderly persons, especially to reduce muscle loss in elderly frailty persons.

WO 2013/021891 A1 discloses novel amino acid-containing compositions for enhancing recovery from muscle fatigue after exercise.

### Summary of the invention

Thus, an object of the present invention is to provide a system which results in a prevention or treatment of sarcopenia, or at least results in a reduction of loss of muscle morphology or an improved muscle morphology.

While, elderly muscles are characterised by having lost strength, function and mass because of an anabolic resistance to meals and a reduced muscle protein synthetic response to dietary amino acids, it may be of serious concern to the individual elderly person and to the society to take of these persons as they can have trouble in taking care of them self. However, without being bound by any theory, the inventors of the present invention believe that it is possible to reduce the muscle loss of elderly people through a combined approach of specific nutrition supplementation with an amino acid sourceselected from whey protein, at least one fatty acid and at least one antioxidant being at least one polyphenol, and electrical muscle stimulation.

Without being bound by any theory, the inventors of the present invention has surprisingly found that when feeding an elderly with a diet or supplement comprising an amino acid source selected from whey protein, at least one fatty acid and at least one antioxidant being at least one polyphenol in combination with electrical muscular stimulation of the elderly's muscles, the loss of muscle mass, size, strength, and function is reduced as compared to when the elderly is exposed to electrical muscle stimulation, but fed with a diet not supplemented with an amino acid source. In fact, the inventors of the present invention have found that feeding an elderly with a diet or supplement comprising an amino acid source selected from whey protein, at least one fatty acid and at least one antioxidant being at least one polyphenol in combination with electrical muscular stimulation of the elderly's muscles, an improvement of muscle mass is obtained as compared to when the elderly person have only received either electrical muscular stimulation or an amino acid source rich diet or supplement.

Further, the inventors of the present invention has surprisingly found out that the administration of a nutritional composition comprising an amino acid source selected from whey protein, at least one fatty acid in combination with an antioxidant being at least one polyphenol to elderly persons in combination with electrical muscular stimulation of the elderly persons, the reduction in loss of muscle mass, size, strength and function is further improved as compared to when elderly persons are fed with a an amino acid source supplement only, and receiving electrical muscle stimulation.

In addition, the inventors of the present invention has surprisingly found out that if elderly persons is given a diet which besides from being rich in an amino acid source selected from whey protein, and at least one antioxidant being at least one polyphenol, comprises essential fatty acids, especially n-3 unsaturated fatty acids, in combination with electrical muscle stimulation, the loss of muscle morphology is further reduced as compared to when elderly persons are fed with a diet rich in an amino acid source only and receiving electrical muscle stimulation.

Thus, one aspect of the invention relates to a composition comprising an amino acid source selected from whey protein, at least one fatty acid and at least one antioxidant being at least one polyphenol for use in combination with electrical muscular stimulation for the treatment or prevention of sarcopenia, for reducing the loss of muscle morphology, for increasing the muscle morphology and/or for improving the muscle recovery after muscle atropy in elderly humans.

Another aspect of the present invention relates to the use of a composition comprising an amino acid source selected from whey protein, at least one fatty acid and at least one antioxidant being at least one polyphenol in combination with electrical muscular stimulation for the treatment or prevention of sarcopenia, for reducing the loss of muscle morphology, for increasing the muscle morphology and/or for improving the muscle recovery after muscle atrophy in elderly humans.

The present disclosure also describes a method of treating an elderly human for sarcopenia, and/or reducing the loss of muscle morphology and/or increasing the muscle morphology and/or for improving the muscle recovery after muscle atrophy, the method comprises i) administration of a composition comprising an amino acid source selected from whey protein, at least one fatty acid and at least one antioxidant being at least one polyphenol to the elderly person and ii) expose the elderly person to electrical muscle stimulation.

The present invention will now be described in more detail in the following.

### Detailed description of the invention

### Definitions

Prior to discussing the present invention in further details, the following terms and conventions will first be defined:

Numerical ranges as used herein are intended to include every number and subset of numbers contained within that range, whether specifically disclosed or not. Further, these numerical ranges should be construed as providing support for a claim directed to any number or subset of numbers in that range. For example, a disclosure of from 1 to 10 should be construed as supporting a range of from 1 to 8, from 3 to 7, from 1 to 9, from 3.6 to 4.6, from 3.5 to 9.9 and so forth. All references to singular characteristics or limitations of the present invention shall include the corresponding plural characteristic or limitation, and vice versa, unless otherwise specified or clearly implied to the contrary by the context in which the reference is made.

In the context of the present invention, the term "ratio" by weight (weight/weight) refers to the ratio between the weights of the mentioned compounds. For example, a mixture comprising 60 g whey protein and 2 g polyphenol would have a weight ratio which is equal to 60:2, which is equal to 30:1 or 30 (that is 30 divided with 1). Similarly, a mixture of 50 g whey protein and 20 g polyphenol would have a ratio by weight of whey protein and polyphenol of 50:20, which is equal to 5:2 or 2.5 (that is 5 divided with 2).

The term "and/or" used in the context of the "X and/or Y" should be interpreted as "X", or "Y", or "X and Y".

All percentages and ratios are by weight unless otherwise specified.

Mentioned percentages pertain to listed ingredients and are based on the active level and, therefore, do not include solvents or by-products that may be included in commercially available materials, unless otherwise specified.

Unless defined otherwise, all technical and scientific terms used herein have the same meaning as commonly understood by one of ordinary skill in the art.

The term "elderly" will in the context of the present invention mean a person above the age of 60 years, such as above 63 years, in particular above 65 years.

In the context of the present invention, the elderly human may be any elderly person, such as a person of both sexes, i.e. both male and female.

In an embodiment of the invention, the elderly humans are frail elderly humans.

In the context of the present invention, the term "frail" referres to a person which is physically weak, i.e. not strong, but fragile.

The composition of the present invention, including the many embodiments described herein, can comprise, consist of, or consist essentially of the essential elements and limitations of the invention described herein, as well as any additional or optional ingredients, components, or limitations described herein or otherwise useful in a diet for elderly persons.

The term "treatment" or "treating" according to the present invention includes any effect, e.g. lessening, reducing, modulating, or eliminating, that results in the improvement of the condition, disorder, etc. "Treating" or "treatment" of a disorder state including: (I) inhibiting the disorder state, i.e. arresting the development of the disorder state or its clinical symptoms; or (2) relieving the disorder state, i.e. causing the temporary or permanent regression of the disorder state or its clinical symptoms.

The term "prevention" or "preventing" as used herein means preventing the disease state, i.e. causing the clinical symptoms of the disease state not to develop in an individual that may be exposed or predisposed to the disease state, but does not yet experience or display symptoms of the disease state.

The term "disorder state" means any disease, condition, symptom, or indication.

In the context of the present application, the term "in combination with" as in a composition comprising "x" in combination with "y" refers to administration in both a single dosage form as one nutritional composition containing both "x" and "y", such as for example an amino acid source, antioxidant and/or fatty acid, as well as the administration in separate dosage forms of the amino acid source, antioxidant, and/or fatty acid, either simultaneously or sequentially.

### Amino acid source:

The amino acid source is a whey protein.

The composition is to be administered in an amount corresponding to 0.03 to 0.5 g amino acid per Kg body weight.

The amino acid source comprises one or more of branched chain amino acids. For example, the amino acid source comprises one or more of leucine, isoleucine, and valine. Leucine may be present as D- or L-leucine and preferably the L-form.

The amino acid source comprises a branched chain amino acid.

The three branched chain amino acids (BCAAs), leucine, valine, and isoleucine, share common enzymes for the first 2 degradative steps, transamination and subsequent decarboxylation, and are also the only indispensable amino acids to have degradative metabolic pathways active in muscle. Therefore, it could be hypothesized that by giving a large dose of a single BCAA (e.g. leucine), it may cause the decarboxylation/oxidation or the other two BCCAs (e.g. isoleucine and valine), causing them to become limiting for muscle protein synthesis - especially in situations where leucine may be "spiked". Indeed, a study by Verhoeven and collegues (Am J Clin Nutr. 2009 Mat;89 (5): 1468-75), also found that leucine supplemention resulted in an approximate 15 and 25% decrease in isoleucine and valine, respectively. In this context, it will be beneficial to add valine and isoleucine in addition to leucine into the composition, thus avoiding a depletion of valine and isoleucine circulating concentration secondary to leucine level.
For this reason, it is not recommended and may be harmful to administer large amounts of leucine which may induce the decrease of other branched amino acids i.e. valine and isoleucine. Thus, leucine should be added in an amount having an remarkable effect on muscles but so low that depletion of valine and isoleucine is avoided. It is recommended to use a maximum dose of leucine of 10 wt% of the dry matter of the composition.

If the amino acid source comprises leucine, the composition is to be administered in an amount corresponding to about 0.03 to 0.2 g leucine per kg body weight. Besides from leucine, the composition may comprise valine and/or isoleucine and preferably also other amino acids.

In an embodiment of the invention, the composition comprises an amino acid source being whey protein in an amount of from 1-95% amino acid source based on dry weight, preferable from 2-90% amino acid source, such as from 3-80% amino acid source, preferably 5-70% amino acid source based on dry weight.

### Whey protein:

In the present invention, whey protein is used as the amino acid source. The whey protein may be unhydrolyzed or hydrolyzed whey protein. The whey protein may be any whey protein, for example the whey protein is one or more selected from the group consisting of a whey protein concentrate, whey protein isolates, whey protein micelles, whey protein hydrolysates, acid whey, sweet whey or sweet whey from which the caseino-glycomacropeptide has been removed (modified sweet whey) or a fraction of whey protein or in any combination thereof.

In a preferred embodiment, the whey protein is a whey protein isolate.

In another embodiment, the whey protein is modified sweet whey. Sweet whey is a readily available by-product of cheese making and is frequently used in the manufacture of nutritional compositions based on cows' milk.

### Adminstration:

The compositions of the present invention are to be administered in a therapeutically effective dose. The therapeutic effective dose can be determined by the person skilled in the art.

The composition is administered to elderly persons in an amount of the amino acid source sufficient to in combination with electrical muscle stimulation at least partially cure sarcopenia or to reduce the loss of skeletal muscles in said elderly person. An amount to adequate or accomplish this is defined as "a therapeutically effective dose". Amounts effective for this purpose will depend on a number of factors known to those of skill in the art such as the severity of the disease and the weight and general state of the elderly person.

The composition may also be administered to elderly persons in an amount sufficient to prevent or at least partially reduce the risk of developing sarcopenia in instances where the person has lost muscle mass, but the condition of sarcopenia has yet not been developed. Such an amount is defined to be "a prophylactically effective dose". Again, the precise amounts depend on a number of factors relating to the elderly persons, such as their weight, health and how much muscle mass is being lost.

The composition comprising whey protein is preferably given as a supplement to an elderly persons diet daily or at least twice a week. The composition is preferably administered to the elderly after electrically muscle stimulation.

The composition of the present invention is most effective when it is administered consecutively for a number of days, ideally until muscle increase/improvement is achieved.

The composition of the present invention should be administered daily for a period of time of at least 30, 60 or 90 consecutive days in combination with the muscle stimulation.

The composition according to the present invention may in combination with muscle stimulation be administered to an elderly person as a supplement to the normal diet for longer period, such as for a period of 1, 2, 3, 4, 5, 6, 7, 8, 9, or 10 years.

The composition of the present invention is ideally administered in a period of at least 3 months in order to see a benefit. Preferred, the composition is administered in a periode of 3 months to 1 year, but it could be for a longer time. Even more preferably the composition is administered for at least 6 months. Administration of the composition to an elderly person for the rest of that persons life may be possible, but preferably the period of administration is 3 to 6 months, such that the elderly person will not be tired of it. Administration for a period of time, such as 3 to 6 monts do not mean a continuously administration every day with no interruptions. There may be some short breaks in the administration, such as a break of 2-4 days during the period of administration.

The ideal duration of the administration of the composition of the present invention may be determined by those of skill in the art.

The composition of the present invention is effective when it is ingested. Hence, in a preferred embodiment the composition is administered orally or enterally, for example via tube feeding.

The ideal duration of the administration of the composition of the present invention may be those of skill in the art.

In preferred embodiments, the composition of the present invention is administered in the form of a beverage, a capsule, a tablet, a powder or a suspension. In an embodiment the composition is an oral nutritional supplement (ONS), a complete nutritional formula, a phamacetutical, a medical or a food product. In a preferred embodiment of the invention the composition comprising an amino acid source, e.g. whey protein, according to the present invention is administered as a beverage. The composition composition may also be stored in a sachet and upon use suspended in water.

In some instances where oral or enteral administration is not possible or not advised, the composition of the present invention may also be administered parenterally.

The composition of the present invention may be any kind of composition that is suitable for human and/or animal consumption.

For example, the composition may be selected from the group consisting of food compositions, dietary supplements, nutritional compositions, nutraceuticals, powdered nutritional products to be reconstituted in water or milk before consumption, food additives, medicaments, beverages and drinks.

In an embodiment of the invention, the composition is a nutritional supplement which is almost entirely made up of whey protein. Thus, in a preferred embodiment, the composition comprises more than 60% whey protein based on dry weight, such as above 70% whey protein, preferably above 80% whey protein, such as above 85% whey protein, even more preferably above 90% whey protein, such as above, 92% whey protein, in particular above 95% whey protein, such as above 97% whey protein based on dry weight.

In another embodiment of the invention, the composition is a nutritional supplement comprising an amino acid source, selected from whey protein, at least one fatty acid, and at least one antioxidant, selected from at least one polyphenol, but also other ingredients optimal for an elderly person to intake, such as one or more of dietary fibres, vitamins, minerals and probiotics. In an embodiment of the invention, the composition comprises whey protein in an amount of from 0.5-50% based on dry weigh, such as from 1-40% whey protein based on dry weight, preferable from 2-35% whey protein, such as from 3-30% whey protein, preferably 5-20% whey protein based on dry weight.

The amount of amino acid source administered to the elderly persons depends of both the weight and health of the elderly person, i.e. the severity of sarcopenia and/or degree of muscle loss in said elderly person.

In an embodiment of the present invention, the composition is to be administered in an amount corresponding to about 0.03 to 1.0 g whey protein per kg body weight per day, such as about 0.05 to 0.7 g whey protein per kg body weight per day, preferably about 0.1 to 0.5 g whey protein per kg body weight per day.

Thus, in an embodiment of the invention the composition comprises whey protein in an amount such that the intake of whey protein is 5-50 g whey protein per day, such as from 12-40 g whey protein per day, preferably from 15-30 g whey protein per day, such as from 16-25 g whey protein per day, even more preferably 20 g whey protein per day.

In an embodiment of the invention, the composition is to be administered in an amount corresponding to about 5-50 g whey protein, such as 10-40 g whey protein, preferably 12-35 g whey protein, such as 15-30 g whey protein.

When an elderly person is consuming the composition according to the present invention in combination with receiving electrical muscle stimulation, a synergistic effect is obtained with regard to reducing muscle atrophy, reducing loss of muscle morphology and to treat or prevent sarcopenia.

In the context of the present application, the term "reduced" in "reduce loss of muscle morphology" refers to that the muscle (size, mass, strength functionality) is at least 0.5% larger than the muscle as compared to. For example, the loss of muscle size is reduced by 0.5%, if the muscle size of a person fed with a composition comprising whey protein and subjected to electrical muscle stimulation is 0.5% larger than the muscle of a person only being subjected to electrical muscle stimulation, but not having received a supplement of whey protein.

In an embodiment of the invention, the term "reduced" in "reduce loss of muscle morphology" means that the muscle is at least 0.8% larger than the muscle compared to which has not been treated with the composition of present invention and receiving EMS, such as at least 1.0% larger, for example at least 1.5% larger, preferably at least 2.0% larger, such as at least 2.5% larger, even more preferably at least 3.0% larger, such as at least 4.0% larger, preferably at least 5.0% larger.

In the context of the present application, the term "increased" in "increased muscle morphology" refers to that the muscle (size, mass, strength, functionality) is at least 0.5% larger than the muscle as compared to. For example, the muscle size is increased by 0.5%, if the muscle size of a person fed with a composition comprising whey protein and subjected to electrical muscle stimulation is 0.5% larger than the muscle of a person only being subjected to electrical muscle stimulation, but not having received a supplement of whey protein.

In an embodiment of the invention, the term "increased" in "increased muscle morphology" means that the muscle is at least 0.8% larger than the muscle compared to which has not been treated with the composition of present invention and receiving EMS, such as at least 1.0% larger, for example at least 1.5% larger, preferably at least 2.0% larger, such as at least 2.5% larger, even more preferably at least 3.0% larger, such as at least 4.0% larger, preferably at least 5.0% larger.

### Antioxidants:

The composition comprises an amino acid source selected from whey protein in combination with at least one antioxidant being at least one polyphenol, and at least one fatty acid.

Without being bound by any theory, the inventors of the present invention have surprisingly found that the effect of electrical muscle stimulation, to reduce sarcopenia is improved when exposed to an elderly person being fed with a nutritional composition comprising an amino acid source and at least one antioxidant, i.e. reduce the loss of muscle morphology, such as loss of muscle mass, size, strenght or function or to improve the muscle morphology, such as the mass, size, strength and function.

The antioxidant is selected from the group of polyphenols.

Preferred are food grade polyphenols. A compound is considered food-grade if it is generally accepted and considered safe for food applications.

Mixtures of antioxidants may be used. For example antioxidants may be provided as food compositions that are known to be rich in antioxidants or as extracts thereof.

In a preferred embodiment the antioxidant is selected from the group consisting of curcumin, green tea catechins, rutin, or combinations thereof.

In another preferred embodiment of the invention, the at least one antioxidant is a combination of two or more antioxidants.

Cocoa, coffee or tea are high in antioxidants.

Several spices or herbs may also be used such as oregano, cumin, ginger, garlic, coriander, onion, thyme, marjoram, tarragon, peppermint, and/or basil.

Fruit extracts or dried fruits may be used. Examples are pears, apples, raisins, grapes, figs, cranberries, blueberries, blackberries, raspberries, strawberries, blackcurrants, cherries, plums, oranges, mango, and/or pomegranates.

As vegetables high in antioxidants cabbage, broccoli, bettroot, artichoke heads, black olives, black beans, celery, onion, parsley and spinach may be mentioned.

Antioxidants may also be used as purified compounds or partially purified compounds.

In an embodiment of the invention, the composition comprising an amino acid source and the at least one antioxidant is in a weight ratio of 40:1 to 1:1, such as from 35:1 to 2:1, preferably form 30:1 to 5:1, such as from 28:1 to 8:1, even more preferably from 25:1 to 10:1.

In the invention, the antioxidant is one or more polyphenol.

### Polyphenols:

In the invention, the composition comprising an amino acid source as defined before is in combination with at least one polyphenol and at least one fatty acid as defined above.

Mixtures of polyphenols may be used. For example, two or more polyphenols may be used. Polyphenols may also be provided as food compositions that are known to be rich in polyphenols or extracts thereof.

Cocoa, coffee and tea are high in polyphenols.

Fruit extracts or dried fruits may be used as a source of polyphenols. Examples are pears, apples, grapes, cranberries, blueberries, blackberries, raspberries, strawberries, blackcurrants, cherries, plums, and/or pomegranates.

Also some nuts and seeds are rich in polyphenols, such as chestnuts, hazel nuts and flaxseed.

Examples of vegetables high in polyphenols are cabbage, broccoli, beetroot, artichoke heads, black olives, black beans, celery, onions, parsley and spinach.

Polyphenols may also be used as purified compounds or partially purified compounds.

Examples of polyphenols are phenolic acids, flavonoids, such as flavonols, flavones, isoflavones, flavanones, anthocyanins, and flavanols, stilbenes and lignans.

In an embodiment according to the invention, the at least one polyphenol may be selected from the group of hesperetine-7-glucoside, curcumin, quercetin, gree tea catethins, and rutin.

In a preferred embodiment, the at least one polyphenol are selected from the group of curcumin, rutin and quercetin.

In a preferred embodiment of the invention, the at least one polyphenol is curcumin.

In another preferred embodiment of the invention, the at least one polyphenol is rutin.

In still another preferred embodiment of the invention, the at least one polyphenol is a combination of two or more polyphenols, for example a combination of rutin and curcumin.

In an embodiment of the invention, the composition comprises whey protein and at least one polyphenol in a weight ratio of 300:1 to 2:1, such as from 100:1 to 5:1, preferably form 60:1 to 10:1, even more preferably form 50:1 to 20:1.

### Vitamin D:

The composition furthermore may be administrated in combination with vitamin D.

Vitamin D is known to have ab effect on muscle function. The molecular mechanisms og vitamin D action on muscle tissue include genomic and non-genomic effect. Genomic effects are initiated by binding of 1,25-dihydroxyvitamin D3 to its nuclear receptor, which results in changes in gene transcription of messenger RNA and subsequent protein synthesis. Non-genomic effects of vitamin D are rapid and mediated through a membrane-bound vitamin D receptor (VDR).

### Fatty acids:

The composition comprising an amino acid source and at least one antioxidant as defined above is in combination with at least one fatty acid. The fatty acid may be any fatty acid and may be one or more fatty acids, such as a combination of fatty acids.

In the invention, the composition comprising an amino acid source is in combination with at least one antioxidant and at least one fatty acid as in claim 1.

Without being bound by any theory, the inventors of the present invention believes that the effect of electrical muscle stimulation and a diet rich in an amino acid source on reducing loss of muscle morphology in an elderly person, will be further improved if the diet also is rich in fatty acid.

The fatty acid is preferably essential fatty acids, such as the essential polyunsaturated fatty acids, linoleic acid (C18:2n-3) and a-linolenic acid (C18:3n-3). Furthermore, the fatty acids may be the long-chain polyunsaturated fatty acids eicosapentaenoic acid (C20:5n-3) and arachidonic acid (C20:4n-6), and docosahexaenoic acid (C22:6n-3) or any combination thereof.

In a preferred embodiment of the invention the fatty acid is a n-3 (omega 3) or n-6 (omega 6) fatty acid, in particular a n-3 fatty acid

The fatty acid is preferably eicosapentaenoic acid.

The fatty acid may be derived from any suitable source containing fatty acids, such as for example coconut oil, rapeseed oil, soya oils, corn oil, safflower oil, palm oil, sunflower oil or egg yolk. The source of fatty acid is however preferably fish oil.

In some embodiments of the invention, the composition comprising the amino acid source and the antioxidant, vitamin D and/ fatty acids are administered in a single dosage form, i.e. all compounds are present in one product to be given to an elderly person in combination with a meal.

In another embodiment of the invention, the composition comprising the amino acid source and the antioxidant, vitamin D and fatty acid are co-administered in separate dosage forms.

### Anabolic resistance:

The term "anabolic resistance" refers to the inability to increase protein synthesis in response to an increase in amino acids following a meal.

### Muscle atrophy:

The term "muscle atrophy" refers in the present application to the wasting or loss of muscle morphology, i.e. loss of muscle tissue.

The term "muscle morphology" will in the context of the present invention mean any parameter relating to muscles, for example muscle size, muscle mass, muscle strength, muscle function, etc.

Muscle atrophy may be caused by many reasons. For example, it may result from lack of physical activity, such as from immobilization or low physical activity associated with ageing (sarcopenia associated with ageing process), hip-fracture recovery or from several co-morbidities of diseases, such as cancer, AIDS, congestive heart failure, COPD (chronic obstructive pulmonary disease), renal failure, trauma, sepsis, and severe burns, for example. Muscle atrophy may also result from insufficient or inappropriate nutrition or starvation.

Very commonly, muscle atrophy results from disuse or insufficient use of the respective muscle.

The muscle referred to in the present invention is preferably a skeletal muscle. For example, the composition of the present invention may be used in combination with electrical muscle stimulation to reduce the loss of muscles in the arms and/or legs. The muscle may be one of the following; gastrocnemius, tibialis, soleus, extensor, digitorum longus (EDL), biceps femoris, semitendinosus, semimembranosus, gluteus maximus or combinations thereof.

In the present invention muscle atrophy may result in the disorder state sarcopenia, i.e. lost muscle mass, size, strength and functionality because of ageing.

The muscle atrophy may be of different grades, such as severe muscle atrophy as in extreme frailty elderly persons. These elderly persons will have difficulty in carry on every day activities and taking care of them self. Muscle atrophy, but of a less severe degree will allow some movement and some muscle activity, but insufficient to sustain the complete muscle tissue.

The cause of sarcopenia are multifactorial and can include disuse, changing endocrine function, chronic diseases, inflammation, insulin resistance and nutritional deficiencies (Fielding et al, J. Am Med. Dir. Assoc. 2011, 12:249-256).

Sarcopenia as an age-associated loss of skeletal muscle mass and function have been discussed in reports. For example, studies have shown that the addition of leucine to the meal of elderly sarcopenia patients may be beneficial to treat sarcopenia. Recent work from Kastanos et al (Am J. Physiol. Endocrinol. Metab. 291:E381-E387, 2006) has clearly shown that addition of leucine in the diet did not increase muscle protein synthesis in young subjects while the same leucine supplementation was efficient in elderly. These authors concluded that elderly people exhibit a decrease sensitivity of muscle protein synthesis to leucine that is not observed in young adults.

Thus, the mechanisms involved in treating or preventing age-associated sarcopenia are different from treating or preventing muscle mass loss in young persons.

Even though, studies have shown that supplementation of leucine to elderly have a beneficial effect to sarcopenia, there still is a need to further improve the treatment of prevention of sarcopenia.

The inventors of the present invention have surprisingly found out that supplementation of the diet of an elderly person with an amino acid source in combination with electrical muscle stimulation of the elderly, the loss of muscle mass, size, strength and function will be further reduced.

### Electrical muscle stimulation:

In the context of the present invention, electrical muscle stimulation (EMS) may also be referred to as muscular electro-stimulation, muscular electric stimulation, neuromuscular electrical stimulation (NMES) or electromyostimulation. These terms may be used interchangeable. Electrical muscle stimulation is the elicitation of muscle contraction using electric impulses. The impulses are generated by an apparatus and delivered through electrodes on the skin in direct proximity to the muscles to be stimulated. The impulses mimic the action potential coming from the central nervous system, causing the muscles to contract.

In an embodiment of the invention, the electrodes are pads which adhere to the skin, but the electrodes may also be other forms.

EMS exercise is preferably given to the elderly person at least once a week, preferably at least two times a week, such as at least three times a week.

The apparatus for physical stimulation or electrically muscle stimulation may be an apparatus or machine forcing muscular functions to enhance energy loss.

The motor unit recruitment in normal voluntary movement is sequential recruitment starting from slow-twitch fiber which is low in contraction tension and slow in exhausting. In previous study, it was shown by electrophysiology that EMS sequentially recruited from fast-twitch fiber controlled by thick nerve fiber. Existence of an energy metabolic property different from that of voluntary contraction was suggested (Hamada et al. 2004; Moritani et al. 2005). It was recently shown, using euglycemic hyperinsulinemic clamp technique and an Expired gas analysis, that EMS accelerated glucose and energy metabolism. In particular, oxygen consumption during EMS was doubled compared with that observed at rest. In the same time, energy consumption was increased (50 kcal by EMS for 20 min).

Thus, it was demonstrated that EMS made possible recruitment of fast-twitch fiber and activation of muscle energy consumption, glycogen metabolism, and glucose metabolism. Moreover, selective stimulation of fast-twitch fiber has the potential of not only preventing the disuse of muscle atrophy in elderly persons, but also the initiation of muscular hypertrophy. These findings suggests that EMS has the potential of producing improvement in metabolisms in people who need exercise limitations due to their physical conditions such as being bed-ridden elderly people and patients who suffer from organ damage caused by diabetic complications and cardiovascular complications. Further, studies on EMS and its health benefits are expected from the point of views of care prevention, preventive medicine, and care medicine. A trial model of leggings with electrodes enabled stimulation of the gluteus maximus muscle and succeeded in initiating muscle contraction of about 6 METS of exercise intensity.

Taken together, the present invention proposes a double approach combining electrical muscle stimulation, and a specific dietary supplementation which is shown to have a positive influence on the quality of life of elderly people and related health economics, as a new method of prevention, improvement, and treatment of lifestyle related diseases caused by aging and lack of exercise.

It should be noted that embodiments and features described in the context of one of the aspects of the present invention also apply to the other aspects of the invention.

Further aspects of the present invention are given as the following items:
1. Use of a composition comprising an amino acid source selected from whey protein, at least one fatty acid and at least one antioxidant being at least one polyphenol in combination with electrical muscular stimulation for the treatment or prevention of sarcopenia, for reducing the loss of muscle morphology, for increasing the muscle morphology and/or for improving the muscle recovery after muscle atrophy in elderly humans.

It should be noted that embodiments and features described in the context of one of the aspects of the present invention also apply to the other aspects of the invention.

All patent and non-patent references cited in the present application, are hereby incorporated by reference in their entirety.

The invention will now be described in further details in the following non-limiting examples.

### Examples

The following examples illustrate the specific embodiments of the composition for use in combination with electrical muscle stimulation according to the present invention. The examples are given solely for the purpose of illustrating and are not to be construed as limitations of the present invention, as many variations thereof are possible without departing from the spirit of the invention.

### Example 1

Example 1 describes a study conducted to study elderly persons being exposed to electrical muscle stimulation while being fed with a diet including a composition comprising whey protein as compared to a being fed with a diet without whey protein.

The primary objective was to determine the efficacy of the intake of a whey-based supplement enriched or not with fish oil and polyphenols in addition to electrical muscle stimulation (EMS) for improvement of muscle morphology and functionality of frail individuals during 12 weeks of EMS and nutritional intervention.

In particular, it was aimed at demonstrating that the effect of electrical muscle stimulation to elderly persons synergistically increased when the elderly persons were fed with a whey protein based supplement, i.e. synergistically improve the muscle morphology (muscle thickness of the quadriceps femoris, hamstrings muscle group, and triceps surae muscle) or muscle strength of elderly persons. This is measured by ultrasonography of the individuals with a specific focus on lower limb and the thigh and respectively by knee extension strength.

The primary comparison between groups administered with different diets will be the increase in muscle thickness or muscle strength between the group receiving the whey protein supplement containing or not fish oil and polyphenols and the group receiving only a carbohydrate supplement (placebo) (see below for detailled groups description).

### MATERIAL & METHODS

This is a parallel, double-blind, randomized study design, and single center with 3 groups (see below)

Forty-one subjects initially volunteered for this study were all frail elderly people (age 65-90 years) presenting a frailty status according to the long-term care insurance (LCTI) system of Japan. All subjects included in the study were "free-living" people without any support or classified in Care support level 1, Care support level 2, Long-term care level 1 (LTC1), and Long-term care level 2 (LTC2) according to LTCI system. Subject with gait speed between 0.6 and 1.2m/s were included. All subjects were screened by a physician and a care manager, asking orthopedic and medical questions, to determine their suitability to participate in exercise interventions including electrical muscle stimulation.

Subjects were randomized in a 1:1:1 ratio to one of the three study groups receivingsocaloric (95kcal) beverage containing either
1. (A) Group 1, 20g of carbohydrate (maltodextrin glucose syrup 21DE) + placebo capsules (CHO) (n=13)
2. (B) Group 2, 20g of whey protein isolate (Prolacta 95) + placebo capsules (WHEY) (n=15), or
3. (C) Group 3, 20g of whey protein isolate (Prolacta 95) plus rutin capsules (500mg rutin per day)+ w3-FA/curcumin capsules (daily doses: 500mg curcumin and 1.5g w3-FA type NAD supplied by Sofinol) (W-BIO) (n=13).

Four subjects (1 in CHOgroup and 3 in W-BIO group) were excluded from the per-protocol analysis population due to subjects not compliant with the inclusion criteria (n=2) or prematurely withdrawn from follow-up (n=2).

Depending on group assignment, subjects ingested orally 1 of 3 experimental beverages (A-C) dissolved in 220ml of water.

On the days with EMS treatment (2 times a week), this beverage was given just after EMS (maximal stimulation of protein synthesis).

To be able to discriminate the specific effect of the supplement (and not the one from the lunch), EMS was applied as soon as possible on the morning (so that the supplement will be taken at least 1 hour before the meal) or later in the afternoon (at least 2 hours after the end of the lunch).

On the day without EMS treatment, subjects drank the dietary supplement at the same time they were used to drink it when they received EMS. The subjects also ingested 7 capsules per day, 2 during the breakfast, 3 during the lunch and 2 during the diner for the duration of the study: 2 hard capsule containing 500mg of rutin/day or placebo, 5 soft capsules containing a mix of fish oil (1.5g fish oil/day) and curcumin (500mg curcumin per day).

All 41 subjects performed a Mini-Nutritional Assessment (MNA) to evaluate their basal nutritional state at baseline and after 12 weeks.

### Electrical muscle stimulation (EMS) procedures.

All subjects received EMS training 2 times a week during 12 weeks. The subjects were asked to attach belt-type electrodes around the waist and both knees and ankles to stimulate inner muscles as well as the gluteus maximus muscle, the quadriceps femoris, the hamstrings, the triceps surae, and tibialis anterior muscles at 20 Hz (Muscle hypertrophy mode) of stimulus frequency. The stimulation intensity of EMS was regulated to the maximal tolerable level of each individual without discomfort. The EMS training was provided to the subjects for 20 min, 2 times per week for 12 weeks. Each time before the EMS, the physical conditions of the subjects were checked. EMS training was performed in a sitting position at rest to minimize the risk of developing lightheadedness, dizziness, falling, and fainting caused by rapid movement. We used a specially designed muscle stimulator (Auto Tens pro, Homer Ion Co. Ltd., Tokyo, Japan) for EMS training in this investigation. The stimulator current waveform was designed to produce co-contractions in the lower extremity muscle groups at a frequency of 20 Hz with a pulse width of 250 µs. The duty cycle was a 5 s stimulation with a 2 s pause for a period of 20 min. Moreover, we used an exponential climbing pulse to reduce discomfort during muscle stimulation.

High-frequency fatigue induces excessive loss of force, which can be due to electrical propagation failure with a rapid decline in the evoked action potential amplitude. During this period of high-frequency force fatigue, considerably greater force is generated at 20 Hz stimulation

Most of the previous studies reported the efficacy of EMS using very high-frequency (2500 Hz) or high-frequency stimulations (50 or 80 Hz). Eriksson E, et al. (Int J Sports Med 1981; 2:18-22.) showed that muscle enzyme activities, fiber size, and mitochondrial properties in the quadriceps femoris did not change with 50 Hz EMS training sessions over 4-5 weeks.

20 Hz EMS has the potential to elicit more effective muscular improvement (a combined adaptation of neural factors and morphological changes) than high-frequency (50 or 80 Hz) EMS.

### Muscle morphology assessment.

Muscle thickness of the quadriceps femoris, hamstrings muscle group, and triceps surae muscle was assessed by using ultrasonography at baseline, 4 weeks, 8 weeks and 12 weeks of the experimental intervention.

Strong correlations have been reported between muscle thickness measured by B-mode ultrasound and site-matched skeletal muscle mass measured by MRI.

The measurement of muscle thickness by ultrasonography was standardized as follows: All scans were carried out at baseline and every four weeks after treatment. Each subject was examined by the same operator, using a real-time scanner (SSD-900, ALOKA, Tokyo, Japan) with a 5 MHz broadband transducer. A water-based gel was applied to the probe before the imaging procedure. During imaging, the transducer was held perpendicular to the surface of the skin and special care was taken to avoid excessive pressure. The measurement site was at the thickest part of the muscles with standardized procedures using skeletal markers carefully located. The imaging and measurements was performed unilaterally with the subjects in a sitting position for the quadriceps femoris and triceps surae muscles, respectively as these muscles were the main determinant for gait speed. The obtained images were stored on site and the entire data were analyzed later by using National Institute of Health (NIH) Image program. Each imaging data was analyzed in blind fashion as to the subject and date information in order to avoid any experimental bias. The measurement of muscle volume by ultrasonography was also standardized in the following way; in addition to the measurement of muscle thickness, we estimated the changes in thigh and calf muscle groups by measuring the circumferences of these muscle groups with standardized procedures. Subcutaneous fat thickness at four sites of each muscle group were determined by ultrasonography and averaged. Then, each muscle group volume was calculated algebraically by using the method of Moritani et al. ( Am J Phys Med 1979; 58:115-130).

Further, the elderly persons were measured at baseline, and at the end of the 12 weeks by the following parameters:
1. i) Physical performance, i.e. walking speed (gait speed) evaluated as the time to walk 6 meters. Gait speed was evaluated three times for each subject and averaged
2. ii) Muscle strength, evaluated by maximal force of knee extension as described in Watanabe K, et al. J Electromyogr Kinesiol 2012a;22:251-8 and Watanabe et al., Res Clin Practice 2012b; 97:468-473
3. iii) Body composition measured by bioelectrical impedance analysis, i.e. measure of fat mass, lean mass, expressed as Kg and %) These measurements were performed using the device (Tanita BC-118D, Tanita Ltd., Tokyo, Japan), using hand-held leads together with flat foot sole electrodes with an excitation current of 500 microamperes at 50 KHz. The impedance value measured was used to calculate the lean mass and fat mass of Arms, Legs, Torso, and Whole body, respectively.
4. iv) Blood test to measure
   1. a. Inflammatory biomarkers (concentrations of acute phase proteins and cytokines)
   2. b. Marker of insulin sensitivity
   3. c. Blood chemistry profile including safety paramenters
   Blood was sampled from an antecubital vein into vacuum tubes after overnight fasting. Blood test for markers of insulin sensitivity (change in blood glucose, hemoglobin A1C, plasma insulin and C-peptide concentrations), red blood cell count, white blood cell count, plasma fatty acid profile, markers of inflammation (CRP, transthyretin, fibrinogen and orosomucoid), blood chemistry of CPK, HDL and LDL Cholesterol, albumin, total protein and triglycerides, respectively. Blood test of coagulation parameters (platelet count, prothrombin time and partial prothrombin time) were measured at baseline, 2, 6, 12 weeks to guarantee safety with the nutritional intervention. For the other parameters, measurements were performed at baseline and after 12 weeks of the intervention with EMS and nutrition supplementation.
5. v) ECG (Heart rate variability (HRV) power spectral analysis is a well-accepted, useful and noninvasive method, and has provided a comprehensive, quantitative and qualitative evaluation of neuroautonomic function under various research and clinical settings.

### RESULTS

At week 12, there was a statistically significant difference for the knee extension strength between W-BIO group and WHEY group (4.17 kg [95% confidence interval (CI) 0.41 - 7.94], p=0.0308) and between W-BIO group and CHO group (5.89 kg [95% confidence interval (CI) 1.78 - 10.01], p=0.0063). For the right knee extension at week 12, there was a statistically significant difference between group 3 and group 1 (5.35 kg [95% confidence interval (CI) 1.13 - 9.57], p=0.0145).

These data suggest that a double approach combining EMS and specific dietary supplementation have a positive influence on muscle strength, and may improve the quality of life of elderly people. Thus, combining EMS and specific dietary intervention may be considered as a new method treatment of lifestyle related diseases caused by aging and lack of exercise.

Until now, no studies have been carried out investigating the combined approach of nutrition and EMS. Currently we hypothesize that the effect of EMS can be improved with the intake of a whey-based nutritional intervention. Additionally, we hypothesize that a specific blend of ingredients containing polyphenols (exhibiting antioxidant effect) and Long Chain Polyunsaturated Fatty Acids (PUFA; demonstrating anti-inflammatory activity and potentially improving muscle insulin sensitivity) will further enhance the benefits of the whey-based nutritional intervention.

Table 1 represents population description of the parameters at baseline. The majority of subjects randomized in the trial were females. Subjects included in group W-BIO tended to be slightly heavier (BMI of 22.7 kg/m2 vs. 20.3 and 21.3 in group CHO and WHEY respectively) and had a larger calf muscle (cross section area, mm2) compared to subjects included in groups CHO and WHEY. The right and left knee extension strength was inferior for subjects randomized to group CHO compared to measurements obtained for subjects randomized to groups WHEY and W-BIO respectively.

**Table 1. Population description of the parameters at baseline.**

| | **CHO (N=13)** | **WHEY (N=15)** | **WHEY-BIO (N=13)** |
|---|---|---|---|
| **Age at ( years ) randomization (years)** | 75.9(8.7) | 78.0(4.9) | 76.6 (7.3) |
| **Body height (cm)** | 151.62 (7.49) | 152.40 (9.42) | 152.77 (8.09) |
| **Body weight (kg)** | 46.98 (10.82) | 49.69 (10.67) | 53.05 (8.81) |
| **Body Mass Index** (**kg**/**m²**) | 20.3 (3.4) | 21.3 (3.5) | 22.7 (3.0) |
| **Thigh (cross section area, mm²)** | 11737.38 (2673.74) | 12213.68 (3190.19) | 12033.29 (2258.83) |
| **Calf (cross section area, mm²)** | 6699.18 (1163.74) | 6853.66 (1767.15) | 7598.23 (1080.07) |
| **Gait speed (m/s)** | 1.20 (0.32) | 1.12 (0.33) | 1.15 (0.45) |
| **Right knee extension strength (kg)** | 20.09 (4.37) | 25.46 (9.59) | 25.03 (13.28) |
| **Left knee extension strength (kg)** | 18.76 (5.53) | 24.85 (8.21) | 22.04 (11.16) |
| Data are mean (SD); N=Number of subjects, %=percentage; SD=standard deviation; min=minimum; max=maximum; perc=percentile | | | |

### Effect of dietary treatment on muscle surface area and thickness at the end of the intervention and along the study (longitudinal analysis)

When the different groups were compared at the end of the treatment period (after 3 months) no statistically significant difference among three groups with respect to the thigh and calf muscle thickness or cross sectional area were found.

By contrast, longitudinal analysis of muscle surface area and thickness measured with the box-plots for the muscle size data stratified by gender and treatment assignment. The plots indicated a systematic increase in muscle size for all treatment groups and for both Males and Females (data not shown). Indeed, the time effect (slope) estimate was positive and statistically significantly different from 0 for Calf cross-sectional area (CSA), Calf thickness, Thigh CSA and Thigh thickness. Globally, results suggests that increase in muscle size occurred uniformly over all treatment groups and both the genders.

Thus, considering together results of muscle morphology reported above, they show that the treatment induced an increase in muscle surface area and thickness which was observed in the 3 groups and thus was probably more related to EMS treatment than to specific nutritional intervention. However, the EMS treatment may have been a facilitator allowing to get the beneficial conditions for the demonstration of the benefit of group 3 (Whey- bioactive) on muscle strength (as described below)

### Muscle strength.

**Table 2 represent changes in Right knee extension strength**

| **Absolute value** | | | | **Change from baseline** | | |
|---|---|---|---|---|---|---|
| | **Group 1 (N=13)** | **Group 2 (N=15)** | **Group 3 (N=13)** | **Group 1 (N=13)** | **Group 2 (N=15)** | **Group 3 (N=13)** |
| **Right knee extension strength (kg)** | | | | | | |
| **Visit 2 (Day 0)** | | | | | | |
| Nr. Available (%) | 13 (100%) | 15 (100%) | 13 (100%) | | | |
| Mean (SD) | 20.09 (4.37) | 25.46 (9.59) | 25.03 (13.28) | | | |
| Median (min-max) | 20.10 (12.1-27.4) | 25.9 (7.27-41.4) | 26.50 (8.32-53.6) | | | |
| [25^{th} -75^{th} perc.] | [18.30-22.10] | [19.00-34.50] | [13.90-32.20] | | | |

| **Visit 8 (Week 12)** | | | | | | |
|---|---|---|---|---|---|---|
| Nr. | 13 (100%) | 15 (100%) | 11 | 13 (100%) | 15 (100%) | 11 |
| Available (%) | | | (84.6%) | | | (84.6%) |
| Mean (SD) | 20.93 (5.55) | 26.93 (9.66) | 29.32 (15.60) | 0.84 (4.20) | 1.46 (3.19) | 2.24 (4.06) |
| Median (min-max) | 22.2 (12.8-29.8) | 26.30 (7.11-42.1) | 32.8 (6.56-60.40) | 0.20 (-6.4-7.20) | 0.40 (-4.3-7.9) | 2.4 (-2.20-11.10 |
| [25th -75th perc.] | [15.1 -23.30] | [22.0 - 32.50] | [125 - 38.10] | [-0.80 - 4.10] | [-0.60-4.30] | [-1.76 - 4.00] |
| N=Number of subjects; Nr. Available = Number of subjects for whom data was available; %=percentage; SD= standard deviation; min= minimum; max= maximum; perc=percentile; kg= kilogram | | | | | | |
| Group 1= EMS + carbohydrate | | | | | | |
| Group 2= EMS + 20g of whey protein isolate | | | | | | |
| Group 3= EMS+ 20g of whey protein isolate+ rutin + curcumin | | | | | | |

**Table 3 represent changes in Left knee extension strength**

| | **Absolute value** | | | **Change from baseline** | | |
|---|---|---|---|---|---|---|
| | **Group 1 (N=13)** | **Group 2 (N=15)** | **Group 3 (N=13)** | **Group 1 (N=13)** | **Group 2 (N=15)** | **Group 3 (N=13)** |
| **Left knee extension strength (kg)** | | | | | | |
| **Visit 2 (Day 0)** | | | | | | |
| Nr. Available (%) | 13 (100%) | 15 (100%) | 13 (100%) | | | |
| Mean (SD) | 18.76 (5.53) | 24.85 (8.21) | 22.04 (11.16) | | | |
| Median (min-max) | 19.10 (10.0-31.10) | 26.40 (12.8-39.20) | 21.20 (6.52-49.5) | | | |
| [25^{th} -75^{th} perc.] | [14.70-21.00] | [17.70-31.00] | [16.10-28.40] | | | |

| **Visit 8 (Week 12)** | | | | | | |
|---|---|---|---|---|---|---|
| Nr. Available (%) | 13 (100%) | 15 (100%) | 11 (84.6%) | 13 (100%) | 15 (100%) | 11 (84.6%) |
| Mean (SD) | 21.08 (4.83) | 26.75 (8.09) | 27.79 (15.14) | 2.32 (4.89) | 1.90 (4.61) | 4.70 (6.94) |
| Median (min-max) | 19.4 (15.7-30.8) | 27.50 (11.9-42.1) | 27.8 (6.93-62.80) | 1.00 (-4.4-10.10) | 2.50 (-7.90-10.40) | 2.0 (-2.00-21.50 |
| [25th -75th perc.] | [17.8 - 24.10] | [22.70 - 32.50] | [15.9 - 32.40] | [-0.50 - 5.00] | [-0.90 - 4.70] | [0.41 - 6.60] |
| N=Number of subjects; Nr. Available = Number of subjects for whom data was available; %=percentage; SD= standard deviation; min= minimum; max= maximum; perc=percentile; kg= kilogram | | | | | | |
| Group 1= EMS + carbohydrate | | | | | | |
| Group 2= EMS + 20g of whey protein isolate | | | | | | |
| Group 3= EMS+ 20g of whey protein isolate+ rutin + curcumin | | | | | | |

Tables 2 and 3 represent the changes of the right and left knee extension muscle strength at the baseline and at week 12 for the three treatment groups, respectively. At week 12, there was a statistically significant difference between W-BIO group and WHEY group (4.17 kg [95% confidence interval (CI) 0.41 - 7.94], p=0.0308) and between W-BIO group and CHO group (5.89 kg [95% confidence interval (CI) 1.78 - 10.01], p=0.0063) for the left knee extension. For the right knee extension at week 12, there was a statistically significant difference between W-BIO group and CHO group (5.35 kg [95% confidence interval (CI) 1.13 - 9.57], p=0.0145).

Moreover, mixed-effects models were fitted in order to take into account the correlation between the two measurements taken on the two knees for each subject at each time point.

### Body composition.

Anthropometric measurements (fat mass, lean mass, express as Kg and %) by using bioelectrical impedance analysis were carried out at baseline and 12 weeks of experimental treatment. There were no statistically significant differences between any of the study treatment groups at the baseline and at week 12.

### Autonomic nervous system activity.

Electrocardiography R-R interval power spectral analyses revealed no statistically significant differences in resting heart rate, LF (sympathetic nervous system activity), HF (parasympathetic nervous system activity) and TP (overall autonomic nervous system activity) between any of the study treatment groups at the baseline and at week 12.

### Blood Analyses

There were no statistically significant differences between any of the study treatment groups for the blood test results for markers of insulin sensitivity (change in blood glucose, hemoglobin A1C, plasma insulin and C-peptide concentrations), red blood cell count, white blood cell count, plasma fatty acid profile, markers of inflammation (CRP, transthyretin, fibrinogen and orosomucoid), blood chemistry of CPK, HDL and LDL Cholesterol, albumin, total protein and triglycerides at baseline and at week 12. Similarly, blood test of coagulation parameters (platelet count, prothrombin time and partial prothrombin time) measured at baseline, 2, 6, 12 weeks demonstrated no significant group differences.

The present preliminary study demonstrated that knee extension strength of frail individuals was significantly increased (18.8% for left leg and 7.3% for the right leg) in the W-BIO group than other two groups (CHO and WHEY) after 12 weeks of bioactive supplement (whey protein, rutin, w3-FA, and curcumin) together with EMS training.

Also this W-BIO group demonstrated the largest improvement in the gait speed (9.6%) among the groups with carbohydrate or whey protein supplementation together with EMS training. Interestingly, this benefit on muscle strength was not related to an increase in muscle size.

The 12 week of antioxidant polyphenol containing bioactive supplementation for the W-BIO group could have potentially maintained muscle fiber compounds to a much better extent than two other groups receiving no such supplement.

Although the measurements of cross-sectional area and muscle thickness of the thigh did not reveal statistically significant differences, but well reserved muscle protein compound could have developed higher contractile force leading to the significantly higher knee extension strength found in the present study. Another possibility to explain the increased muscle strength observed specifically in W-BIO group would be that the polyphenols may help to manage the oxidative stress that is known to occur in inactive elderly persons. As already discussed, frail elderly needs to eat more protein to induce the same postprandial anabolism. This phenomenon is described as an "anabolic resistance". The polyphenols given together with protein may help to reverse the increase stimulation "anabolic threshold" (by managing the oxidative stress) and thus could restore the anabolic effect of nutrients and proteins on muscle protein synthesis. In the same way, the EPA supplementation may also have induced an improvement in insulin sensitivity and thus stimulated muscle protein synthesis associated with whey proteins.

It is quite clear that human voluntary strength is determined not only by the quantity of muscle (muscle cross sectional area) but also by the quality (muscle fiber types) of the involved muscle mass, and but by the extent to which the muscle mass has been activated (neural factors)( Moritani 1992, 1993). Moreover, muscle quality is also related to intramuscular lipid inclusion, which increases with age and is responsible for a low muscle quality. Thus, the modification of muscle quality along the study and particulary to the benefit seen on W-BIO treatment group, could be a decrease in lipid content in muscle together with an increase of muscle fiber number or size, thus allowing to get an improvement in strength, without any modification of muscle thickness or CSA (counterbalance between lipid and protein content).

Taken together, the present study proposes a double approach combining EMS and specific dietary supplementation which may have a positive influence on the quality of life of elderly people and related health economics, as a new method of prevention, improvement, and treatment of lifestyle related diseases caused by aging and lack of exercise.

## Claims

1. A composition comprising an amino acid source for therapeutic use in combination with electrical muscular stimulation for the treatment or prevention of sarcopenia, for reducing the loss of muscle morphology, for increasing the muscle morphology and/or for improving the muscle recovery after muscle atrophy in elderly humans wherein said composition is in combination with at least one antioxidant and at least one fatty acid, wherein the amino acid source is a whey protein, and wherein the at least one antioxidant is at least one polyphenol.

2. The composition according to claim 1, wherein the elderly human or animals are fragile elderly humans.

3. The composition according to claim 2 for use in reducing the loss of muscle mass, reducing the loss of muscle size, reducing the loss of muscle strength, reducing the loss of muscle function, increase the muscle mass, increase the muscle size, increase the muscle strength or increase the muscle function.

4. The composition according to any of the claims 1 to 3, wherein the amino acid source comprises a branched chain amino acid.

5. The composition according to any of the claims 1 to 4, wherein the muscle is a skeletal muscle, for example selected from the group consisting of gastrocnemius, tibialis, soleus, extensor digitorum longus (EDL), biceps femoris, semitendinosus, semimembranosus, gluteus maximus or combinations thereof.

6. The composition according to any of claims 1 to 5, wherein the composition comprises the amino acid source in an amount from 0.2-100% based on dry weight of the composition.

7. The composition according to claim 1, wherein the polyphenol are selected from the group of curcumin, rutin, and quercetin.

8. The composition according to any of the claims 1 to 7, wherein the composition is in combination with vitamin D.

9. The composition according to claims 1 to 8, wherein the weight ratio between the amino acid source and the at least one antioxidant is 40:1 to 1:1.

10. The composition according to claim 1, wherein the fatty acid is a n-3 fatty acid.

11. The composition according to any of the claims 8-10, wherein the amino acid source, antioxidant, vitamin D and/or fatty acid are present in a single dosage form.

12. The composition according to any of the claims 8-11, wherein the amino acid source, antioxidant, vitamin D and/or fatty acid are co-administered as separate dosage forms.

13. The composition according to any one of claims 1 to 12, wherein the composition is selected from the group consisting of food compositions, dietary supplements, nutritional compositions, nutraceuticals, powdered nutritional products to be reconstituted in water or milk before consumption, food additives medicaments, drink, and pet food.

## Patentansprüche

1. Zusammensetzung, umfassend eine Aminosäurequelle zur therapeutischen Anwendung in Kombination mit elektrischer Muskelstimulation zur Behandlung oder Prävention von Sarkopenie, zum Reduzieren des Verlusts der Muskelmorphologie, zum Steigern der Muskelmorphologie und/oder zum Verbessern der Muskelerholung nach Muskelatrophie bei älteren Menschen, wobei die Zusammensetzung in Kombination mit mindestens einem Antioxidationsmittel und mindestens einer Fettsäure vorliegt, wobei die Aminosäurequelle ein Molkenprotein ist und wobei es sich bei dem mindestens einen Antioxidationsmittel um mindestens ein Polyphenol handelt.

2. Zusammensetzung nach Anspruch 1, wobei es sich bei den älteren Menschen oder Tieren um gebrechliche ältere Menschen handelt.

3. Zusammensetzung nach Anspruch 2 zur Verwendung beim Reduzieren des Verlusts von Muskelmasse, beim Reduzieren des Verlusts an Muskelgröße, beim Reduzieren des Verlusts an Muskelkraft, beim Reduzieren des Verlusts an Muskelfunktion, bei der Steigerung der Muskelmasse, bei der Steigerung der Muskelgröße, bei der Steigerung der Muskelkraft oder bei der Steigerung der Muskelfunktion.

4. Zusammensetzung nach einem der Ansprüche 1 bis 3, wobei die Aminosäurequelle eine verzweigtkettige Aminosäure umfasst.

5. Zusammensetzung nach einem der Ansprüche 1 bis 4, wobei der Muskel ein Skelettmuskel ist, zum Beispiel ausgewählt aus der Gruppe bestehend aus Zwillingswadenmuskel, Schienbeinmuskel, Schollenmuskel, langem Zehenstrecker, zweiköpfigem Muskel des Oberschenkels, Halbsehnenmuskel, halbmembranösem Muskel, großem Gesäßmuskel oder Kombinationen davon.

6. Zusammensetzung nach einem der Ansprüche 1 bis 5, wobei die Zusammensetzung die Aminosäurequelle in einer Menge von 0,2-100 %, bezogen auf das Trockengewicht der Zusammensetzung, umfasst.

7. Zusammensetzung nach Anspruch 1, wobei das Polyphenol aus der Gruppe bestehend aus Kurkumin, Rutin und Quercetin ausgewählt sind.

8. Zusammensetzung nach einem der Ansprüche 1 bis 7, wobei die Zusammensetzung in Kombination mit Vitamin D vorliegt.

9. Zusammensetzung nach einem der Ansprüche 1 bis 8, wobei das Gewichtsverhältnis zwischen der Aminosäurequelle und dem mindestens einen Antioxidationsmittel bei 40:1 bis 1:1 liegt.

10. Zusammensetzung nach Anspruch 1, wobei es sich bei der Fettsäure um eine Omega-3-Fettsäure handelt.

11. Zusammensetzung nach einem der Ansprüche 8-10, wobei die Aminosäurequelle, das Antioxidationsmittel, das Vitamin D und/oder die Fettsäure in einer einzigen Darreichungsform vorliegen.

12. Zusammensetzung nach einem der Ansprüche 8-11, wobei die Aminosäurequelle, das Antioxidationsmittel, das Vitamin D und/oder die Fettsäure als getrennte Darreichungsformen gemeinsam verabreicht werden.

13. Zusammensetzung nach einem der Ansprüche 1 bis 12, wobei die Zusammensetzung aus der Gruppe bestehend aus Nahrungsmittelzusammensetzungen, Nahrungsergänzungsmitteln, Nährstoffzusammensetzungen, Nutrazeutika, pulverisierten Nährstoffprodukten, die vor dem Verzehr in Wasser oder Milch aufzulösen sind, Nahrungsmittelzusätzen, Medikamenten, Getränken und Tierfutter ausgewählt ist.

## Revendications

1. Composition comprenant une source d'acide aminé pour utilisation thérapeutique en combinaison avec une stimulation musculaire électrique pour le traitement ou la prévention de sarcopénie, pour réduire la perte de morphologie musculaire, pour augmenter la morphologie musculaire et/ou pour améliorer la récupération musculaire après une atrophie musculaire chez des personnes âgées dans laquelle ladite composition est en combinaison avec au moins un antioxydant et au moins un acide gras, dans laquelle la source d'acide aminé est une protéine de lactosérum, et dans laquelle l'au moins un antioxydant est au moins un polyphénol.

2. Composition selon la revendication 1, dans laquelle la personne âgée ou les animaux sont des personnes âgées fragiles.

3. Composition selon la revendication 2, pour une utilisation de réduction de la perte de masse musculaire, de réduction de la perte de taille musculaire, de réduction de la perte de force musculaire, de réduction de la perte de fonction musculaire, d'augmentation de la masse musculaire, d'augmentation de la taille musculaire, d'augmentation de la force musculaire ou d'augmentation de la fonction musculaire.

4. Composition selon l'une quelconque des revendications 1 à 3, dans laquelle la source d'acide aminé comprend un acide aminé à chaîne ramifiée.

5. Composition selon l'une quelconque des revendications 1 à 4, dans laquelle le muscle est un muscle squelettique, par exemple, choisi dans le groupe constitué de gastrocnémien, jambier, soléaire, extenseur commun des orteils (EDL), biceps fémoral, semi-tendineux, semi-membraneux, grand fessier ou des combinaisons de ceux-ci.

6. Composition selon l'une quelconque des revendications 1 à 5, dans laquelle la composition comprend la source d'acide aminé en une quantité allant de 0,2 à 100 % sur la base du poids sec de la composition.

7. Composition selon la revendication 1, dans laquelle le polyphénol sont choisis dans le groupe de curcumine, rutine et quercétine.

8. Composition selon l'une quelconque des revendications 1 à 7, dans laquelle la composition est en combinaison avec de la vitamine D.

9. Composition selon les revendications 1 à 8, dans laquelle le rapport pondéral entre la source d'acide aminé et l'au moins un antioxydant va de 40:1 à 1:1.

10. Composition selon la revendication 1, dans laquelle l'acide gras est un acide gras n-3.

11. Composition selon l'une quelconque des revendications 8 à 10, dans laquelle la source d'acide aminé, l'antioxydant, la vitamine D et/ou l'acide gras sont présents dans une forme pharmaceutique unique.

12. Composition selon l'une quelconque des revendications 8 à 11, dans laquelle la source d'acide aminé, l'antioxydant, la vitamine D et/ou l'acide gras sont coadministrés en tant que formes pharmaceutiques indépendantes.

13. Composition selon l'une quelconque des revendications 1 à 12, dans laquelle la composition est choisie dans le groupe constitué de compositions alimentaires, compléments diététiques, compositions nutritionnelles, produits nutraceutiques, produits nutritionnels en poudre destinés à être reconstitués dans de l'eau ou du lait avant consommation, médicaments additifs alimentaires, boisson, et aliment pour animal de compagnie.
